# EUROPÄISCHE PATENTSCHRIFT

(11) **EP 3 374 346 B1**
(45) Veröffentlichungstag und Bekanntmachung des Hinweises auf die Patenterteilung: **24.07.2019**
(21) Anmeldenummer: 16794576.5
(22) Anmeldetag: 08.11.2016
(51) Int. Cl.: B01D 3/00, B01D 3/14, B01D 9/00, C07C 303/44

(54) **VERFAHREN ZUR AUFREINIGUNG VON ALKANSULFONSÄUREN**
METHOD FOR PURIFYING ALKANESULFONIC ACIDS
PROCÉDÉ DE PURIFICATION D'ACIDES ALCANESULFONIQUES

(30) Priorität: 10.11.2015 EP 15193899
(43) Veröffentlichungstag der Anmeldung: 19.09.2018
(73) Patentinhaber: BASF SE, 67056 Ludwigshafen am Rhein (DE)
(72) Erfinder: SPIELMANN, Jan, 68199 Mannheim (DE); KOCH, Michael, 67346 Speyer (DE); WORTMANN, Jürgen, 67117 Limburgerhof (DE); RUETHER, Feelly, 67227 Frankenthal (DE); WEIGUNY, Sabine, 67251 Freinsheim (DE); BORGMEIER, Frieder, 68163 Mannheim (DE)
(86) Internationale Anmeldenummer: PCT/EP2016/076958
(87) Internationale Veröffentlichungsnummer: WO 2017/080991

(56) Entgegenhaltungen:
- EP-A1- 0 405 289
- DE-A1- 2 643 000
- DE-A1-102007 020 697
- DE-T2- 69 002 877
- A. BERTHOUD: "QUELQUES PROPRIETES PHYSICO-CHIMIQUE DES ACIDES ETHANE- ET METHANE-SULFONIQUE", HELV. CHIM. ACTA, Bd. 12, 1929, Seiten 859-865, XP002765992, in der Anmeldung erwähnt

## Beschreibung

Die Erfindung geht aus von einem Verfahren zur Aufreinigung von Alkansulfonsäuren.

Alkansulfonsäuren, insbesondere Methansulfonsäure (MSA), werden rein und in Mischung mit Wasser und anderen Lösemitteln in vielfältigen Anwendungen eingesetzt. Besonders verbreitet ist der Einsatz der MSA in der Galvanotechnik, bei der Weißblecherzeugung und bei der Drahtverzinnung. Alkansulfonsäuren werden auch als Lösemittel oder als Katalysator beispielsweise bei Alkylierungs- und Veresterungsreaktionen verwendet. Ein weiteres Einsatzgebiet für Alkansulfonsäuren ist die Herstellung von Biodiesel, bei der die üblicherweise eingesetzte Schwefelsäure durch Alkansulfonsäuren aufgrund deren besserer Anwendungseigenschaften ersetzt werden kann.

Weiterhin sind Alkansulfonsäuren auch eine Alternative zu Phosphorsäure-haltigen Reiniger-Formulierungen. Da insbesondere Methansulfonsäure leicht lösliche Salze bildet und gut biologisch abbaubar ist, kann durch den alternativen Einsatz von Alkansulfonsäure ein Beitrag zum Gewässerschutz geleistet werden.

Im Folgenden werden alle Komponenten der Rohalkansulfonsäure, die verschieden sind von der Alkansulfonsäure und von Wasser, unter dem Begriff "Verunreinigungen" zusammengefasst. Unter dem Begriff "Leichtsieder" werden Wasser und alle Komponenten, die einen Siedepunkt unterhalb dem Siedepunkt der Alkansulfonsäure haben, verstanden. Als "Schwersieder" werden alle Komponenten bezeichnet, deren Siedepunkt oberhalb des Siedepunkts der Alkansulfonsäure liegt. Die Alkansulfonsäure ist insbesondere Methansulfonsäure.

Bei der Herstellung von Alkansulfonsäuren fällt zunächst Rohalkansulfonsäure an. Hierbei handelt es sich um ein Gemisch aus Alkansulfonsäure, Leichtsiedern und Schwersiedern, wobei die Leichtsieder und Schwersieder abhängig vom Herstellverfahren variieren können. Leichtsieder sind im Allgemeinen Wasser, Salpetersäure, Salzsäure, Thioester, Alkansulfonsäurechlorid, Schwefeltrioxid, Alkane und Alkylsulfone. Als Schwersieder sind oftmals Schwefelsäure, Alkandisulfonsäuren oder Chloralkansulfonsäure enthalten. Zudem können auch farbgebende Substanzen enthalten sein.

Zur Gewinnung der reinen Alkansulfonsäure oder von wässrigen Lösungen der Alkansulfonsäure wird die Rohalkansulfonsäure üblicherweise durch Destillation, Nanofiltration, selektive Absorption von Verunreinigungen an Austauscherharzen oder selektive Fällung der Verunreinigungen als Salze aufgereinigt. Dabei ist die Destillation das dominierende Verfahren, wobei eine Strippung als destillatives oder verdampfendes Verfahren betrachtet wird und die Destillation dabei üblicherweise bei Drücken unterhalb des Umgebungsdrucks durchgeführt wird, da bei den zur Destillation unter Umgebungsdruck erforderlichen Temperaturen die Alkansulfonsäure Zersetzungsprodukte bilden kann. Beispielsweise kann sich bei der destillativen Aufreinigung von Methansulfonsäure Methansulfonsäuremethylester bilden. Ein weiteres Problem ist, dass Methansulfonsäure bei den notwendigen hohen Temperaturen korrosiv wirkt und nur eine beschränkte Auswahl an beständigen Konstruktionsmaterialien zur Verfügung steht.

Aus WO-A 00/31027 ist die Herstellung von Alkansulfonsäure durch Oxidation von Alkylmercaptanen, Dialkyldisulfiden oder Dialkylpolysulfiden mit Salpetersäure bekannt. Als Produkte entstehen Stickoxide, Wasser und weitere Nebenprodukte wie Schwefelsäure. Durch Oxidation mit Sauerstoff wird die Salpetersäure aus den Stickoxiden regeneriert und in den Prozess zurückgeführt. Zur Aufreinigung werden Leichtsieder und Schwersieder über eine Destillation in zwei Schritten abgetrennt und so eine reine, praktisch wasserfreie Alkansulfonsäure gewonnen. Wasser und Salpetersäure werden aus dem Rohprodukt in einer als Abtriebskolonne betriebenen Wasserabtrennkolonne bei leichtem Unterdruck abgetrennt. Das Sumpfprodukt enthält dabei 1 Gew.-% Wasser und etwa 1 Gew.-% Schwersieder, vor allem Schwefelsäure. Die Abtrennung der Schwersieder gelingt durch Destillation der Alkansulfonsäuren mit Reinheiten von mehr als 99,5 Gew.-% und Schwefelsäuregehalten von weniger als 50 ppm im hohen Vakuum, das heißt bei einem Druck von 0,1 bis 20 mbar (abs).

In WO-A 2015/086645 wird die Herstellung von Alkansulfonsäure durch Oxidation von Dialkyldisulfiden mit Stickoxiden beschrieben. Die Stickoxide werden beispielsweise mit Sauerstoff angereicherter Luft regeneriert. Danach werden die Reaktionsprodukte über zwei Destillationskolonnen von Leicht- und Schwersiedern befreit. Das so gereinigte Produkt enthält Methansulfonsäure mit einer unspezifizierten Konzentration.

In GB- A 1350328 ist die Synthese von Alkansulfonsäuren durch Chlorierung von Alkylmercaptanen oder Dialkyldisulfiden in wässriger Salzsäure beschrieben. Das Produkt der Reaktion ist eine 70 bis 85 Gew.-%ige Alkansulfonsäure. Zur Herstellung von wasserfreier Methansulfonsäure wird hier ein zweistufiger Prozess beschrieben. Im ersten Schritt wird Wasser abdestilliert und in einem zweiten Schritt die Methansulfonsäure mit einer kurzen Kolonne aus dem Sumpfprodukt herausdestilliert und über Kopf gewonnen.

In WO-A 2005/069751 wird die Synthese von Methansulfonsäure aus Schwefeltrioxid und Methan über eine Radikalkettenreaktion mit beispielsweise Marshall'sche Säure als Radikalstarter beschrieben. In dieser Synthese wird wasserfreie Methansulfonsäure gebildet, es werden jedoch keine Angaben zur Reinigung gemacht. WO-A 2015/071365 beschreibt einen ähnlichen Prozess, wobei zur Reinigung der resultierenden Methansulfonsäure eine Destillation vorgeschlagen wird. Das Produkt dieses Herstellungsverfahrens ist weitgehend frei von Wasser. Es enthält allerdings Schwefeltrioxid.

CN-A 1810780 beschreibt die Synthese von Methansulfonsäure durch Reaktion von Ammoniumsulfit mit Dimethylsulfat. Dabei entstehen Ammoniummethylsulfonat und Ammoniumsulfat.

Durch Zugabe von Calciumhydroxid wird lösliches Calciummethylsulfonat und unlösliches Calciumsulfat gebildet, welches leicht abgetrennt werden kann. Schwefelsäure wird zugegeben um Methansulfonsäure freizusetzen und erneut Calciumsulfat zu bilden und auszufällen. Aus der gebildeten wässrigen Lösung wird durch Destillation zuerst Wasser entfernt und dann Methansulfonsäure durch Destillation im Unterdruck gewonnen.

DE-C 197 43 901 beschreibt die Synthese von Methansulfonsäure durch Reaktion von Sulfitionen mit Dimethylsulfat. Dabei werden Sulfitionen in einem wässrigen System bei erhöhter Temperatur umgesetzt und einer starken Säure ausgesetzt. Als Nebenprodukt fällt Sulfat beispielsweise in Form von Natriumsulfat an. Die Aufreinigung der Säure erfolgt durch Destillation.

EP-A 0 675 107 beschreibt einen Prozess zur kontinuierlichen Herstellung von Alkansulfonsäurechlorid (ASC) oder Alkansulfonsäure (ASA) in dem ein Alkanmercaptan oder ein Dialkandisulfid mit Chlor in wässriger Salzsäure auch bei erhöhtem Druck umgesetzt wird. Chlorwasserstoff (HCl) und andere unter den verfahrenstechnischen Bedingungen nicht kondensierbare Leichtsieder werden nach Entspannung des Überdrucks ausgetrieben. ASC wird im bevorzugten Temperaturbereich von 10 bis 35°C hergestellt und über eine Destillationskolonne gereinigt. ASA wird aus ASC durch Hydrolyse bei über 80°C bis 135°C mit anwesendem Wasser gewonnen. Die Reinigung von ASC und oder ASA wird beispielsweise auch mit einem Dampfstripper durchgeführt, in dem auch Reste von ASC hydrolysiert werden.

Die Entfernung von Wasser aus einer wässrigen Methansulfonsäure durch Evaporation des Wassers in einem Fallfilmverdampfer im Unterdruck ist in US 4,450,047 beschrieben. Wasser wird über Kopf abgezogen und es wird ein Produktstrom mit mehr als 99,5 Gew.-% Methansulfonsäure erhalten.

Aus US 4,938,846 ist die Entfernung von Wasser aus einer wässrigen Methansulfonsäure durch Verdampfung des Wassers in zwei hintereinandergeschalteten Fallfilmverdampfern, die beide im Unterdruck betrieben werden, bekannt.

DE 690 02 877 T2 offenbart ebenfalls ein Verfahren zur Aufreinigung von Alkansulfonsäuren.

Nachteilig bei den nach dem Stand der Technik bekannten Destillationsverfahren ist, dass der Prozess aufgrund der hohen Temperaturen und dem erforderlichen Unterdruck sehr energieaufwändig ist. Zudem können Schwersieder wie Schwefelsäure ohne besonders energieaufwendige Überführung der Alkansulfonsäure in die Gasphase nicht abgetrennt werden. Auch wird bei einigen Reinigungsprozessen die Destillationsaufgabe mit Fallfilmverdampfern gelöst, die im großindustriellen Maßstab nur schwierig einzusetzen sind.

Ein ebenfalls sehr energieintensives Verfahren ist aus US 4,035,242 bekannt, bei dem wässrige Methansulfonsäure in einem zweistufigen Destillationsprozess gereinigt wird. In der ersten Destillationskolonne wird ein Großteil des Wassers im Unterdruck als Leichtsieder entfernt. Das Methansulfonsäure enthaltende Sumpfprodukt wird in einer zweiten Rektifikationskolonne im Unterdruck verdampft und aufgetrennt, wobei die Methansulfonsäure gewonnen wird.

Die Entfernung von Schwefelsäure aus Methansulfonsäure mit Hilfe basischer Anionentauscherharze ist aus US 6,337,421 bekannt. Des Weiteren werden andere Verfahren zur Abtrennung von Schwefelsäure beschrieben, zum Beispiel Destillation oder fraktionierende Kristallisation sowie die Trennung durch Nanofiltration, die gemäß der Beschreibung der US 6,337,421 jedoch alle keine zufriedenstellenden Ergebnisse liefern.

Die Reinigung von Methansulfonsäure, die oxidierbare Verbindungen enthält, ist in EP-A 0 505 692 oder EP-A 0 373 305 beschrieben. Hierbei wird gemäß EP-A 0 505 692 Chlor zugeführt, um die Verunreinigungen in Methansulfonsäurechlorid zu überführen, das in einem weiteren Schritt zu Methansulfonsäure und Salzsäure hydrolysiert wird. Gemäß EP-A 0 373 305 wird Ozon zugeführt, durch das Methylthiosulfat in Methansulfonsäure umgewandelt wird. Diese beiden Verfahren haben jedoch den Nachteil, dass schwersiedende Komponenten wie Schwefelsäure nicht entfernt werden können und daher weitere Reinigungsschritte erforderlich sind.

Die fraktionierende Kristallisation von Methansulfonsäure und auch von Ethansulfonsäure ist aus R. A. Craig et al., J. Am. Chem. Soc., 1950, Vol. 72, Seiten 163 bis 164 oder A. Berthoud, Helv. Chim. Acta, 1929, Vol. 12, Seite 859 prinzipiell bekannt, jedoch geben die dort beschriebenen Verfahren keinen Hinweis auf eine Umsetzung in großindustriellen Herstell- und Reinigungsverfahren.

Aufgabe der vorliegenden Erfindung war es daher, ein Verfahren bereitzustellen, das weniger energieintensiv ist als die bekannten Verfahren und das eine ausreichende Aufreinigung von Alkansulfonsäuren erlaubt und die Abtrennung aller Verunreinigungen ermöglicht und das im großindustriellen Maßstab wirtschaftlich umgesetzt werden kann.

Diese Aufgabe wird gelöst durch ein Verfahren zur Aufreinigung von Alkansulfonsäuren, folgende Schritte umfassend:
(a) Destillation einer Rohalkansulfonsäure enthaltenden Schmelze zur vollständigen oder teilweisen Abtrennung von Leichtsiedern, wobei die Leichtsieder am Kopf einer Destillationskolonne oder eines einstufigen Verdampfungsapparates abgezogen werden und ein Alkansulfonsäure, Schwersieder und Reste an Leichtsiedern enthaltender Stoffstrom am Sumpf der Destillationskolonne oder des einstufigen Verdampfungsapparats entnommen wird,
(b) Zufuhr des Alkansulfonsäure, Schwersieder und Reste an Leichtsiedern enthaltenden Stromes als Ausgangsschmelze in eine Schmelzekristallisation, wobei sich Kristalle der Alkansulfonsäure, von Hydraten der Alkansulfonsäure oder einer Mischung aus beiden suspendiert in Mutterlauge bilden,
(c) Durchführen einer Fest- Flüssig-Trennung, um die Kristalle aus der Mutterlauge abzutrennen,
(d) Gegebenenfalls Waschen der Kristalle, um an den Kristallen anhaftende Mutterlauge zu entfernen.

Überraschenderweise hat sich gezeigt, dass durch die Kombination einer Destillation zur vollständigen oder teilweisen Abtrennung von Leichtsiedern und einer nachfolgenden Schmelzekristallisation Verunreinigungen aus der Alkansulfonsäure entfernt werden können und zudem der notwendige Energiebedarf gegenüber bekannten Verfahren zur Aufreinigung der Alkansulfonsäure reduziert ist. Weiterhin erlaubt das erfindungsgemäße Verfahren sowohl die Herstellung verschiedener Alkansulfonsäuren als auch der Hydrate der Alkansulfonsäuren.

Ein weiterer Vorteil ist, dass anders als bei derzeit eingesetzten Verfahren zur destillativen Aufreinigung der Alkansulfonsäure durch das erfindungsgemäße Verfahren insbesondere bei der Aufreinigung von Methansulfonsäure auf einfache Weise hohe Reinheiten erzielt werden können. So kann beispielsweise der Anteil an Methansulfonsäuremethylester im Produkt beim erfindungsgemäßen Verfahren deutlich verringert werden. Darüber hinaus werden durch das erfindungsgemäße Verfahren auch Schwersieder zum größten Teil aus der Alkansulfonsäure abgetrennt, was nach dem Stand der Technik nur durch energieintensive Destillation mittels Überführung der Alkansulfonsäure in den Gasraum erfolgt.

Die Destillation in Schritt (a) umfasst erfindungsgemäß auch eine einfache Gasabtrennung von Flüssigkeiten, die auch Feststoffe enthalten können. Eine solche Gasabtrennung ist zum Beispiel ein Flash-Prozess, der beispielsweise bei einer Entspannung einer unter Druck stehenden Flüssigkeit auftritt, wenn Leichtsieder nach einer Druckreduktion in den Gasraum übertreten.

Die Destillation kann sowohl in einer Destillationskolonne als auch einem einstufigen Verdampfungsapparat durchgeführt werden. Wenn nachfolgend der Begriff "Destillationskolonne" verwendet wird, ist immer alternativ auch ein einstufiger Verdampfungsapparat, beispielsweise ein Fallfilmverdampfer, ein Dünnschichtverdampfer, ein Rotationsverdampfer oder ein beliebiger anderer dem Fachmann bekannter Verdampfer einsetzbar.

Die in Schritt (a) zugeführte Rohalkansulfonsäure entstammt einem üblichen Verfahren zur Herstellung von Alkansulfonsäuren. Entsprechende Verfahren sind zum Beispiel in der WO-A 00/31027, der GB- A 1350328, der EP-A 0 675 107, der CN-A 1810780 oder der WO-A 2015/071365 beschrieben. Je nach eingesetztem Herstellverfahren enthält die Rohalkansulfonsäure unterschiedliche Leichtsieder und Schwersieder als Verunreinigungen.

Typischerweise in der Rohalkansulfonsäure, insbesondere Rohmethansulfonsäure, enthaltene Leichtsieder sind zum Beispiel Wasser, kurzkettige Kohlenwasserstoffe mit 1 bis 8 C-Atomen, kurzkettige Alkohole mit 1 bis 8 C-Atomen, Salpetersäure, Salzsäure, Chlor, Alkylmercaptane, Dialkyldisulfide, Dialkylpolysulfide, Ester, beispielsweise Methansulfonsäuremethylester, teil- und vollchlorierte Alkansulfonsäuren, Methansulfonsäurechlorid, Schwefeldioxid, Ammoniak, Dimethylsulfat, Monomethylsulfat und Dimethylsulfon. Bei dem in der WO-A 00/31027 beschriebenen Verfahren fallen üblicherweise Wasser, Alkylmercaptane, Dialkyldisulfide, Dialkylpolysulfide, Alkylalkanthiosulfonate wie Methylmethanthiosulfonat, Alkylalkanthiosulfinate, Dialkyldisulfoxide, C₁-Cₓ-Alkohole, Salpetersäure, Stickstoffoxide, Methansulfonsäuremethylester und Schwefeldioxid als Leichtsieder an. Bei dem in der GB- A 1350328 beschriebenen Verfahren sind übliche Leichtsieder Wasser, Alkylmercaptane, Dialkyldisulfide, Dialkylpolysulfide, Alkylalkanthiosulfonate wie Methylmethanthiosulfonat, Alkylalkanthiosulfinate, Dialkyldisulfoxide, Alkansulfonylhalogenide wie Methansulfonylchlorid, Halogene wie Chlor oder Brom, Halogenwasserstoffe wie Chlorwasserstoff oder Bromwasserstoff, C-halogenierte Methansulfonsäure-Verbindungen, Methansulfonsäuremethylester und Schwefeldioxid. Bei dem in der CN-A 1810780 beschriebenen Verfahren sind üblicherweise enthaltene Leichtsieder Wasser, Ammoniak, Methanol, Dimethylsulfat, Monomethylsulfat, Schwefeldioxid und Methansulfonsäuremethylester. Bei den nicht wasserfrei arbeitenden Herstellverfahren bildet in der Regel jeweils Wasser den größten Anteil der Leichtsieder.

Bei wasserfrei arbeitenden Herstellverfahren für Alkansulfonsäuren sind übliche Leichtsieder beispielsweise kurzkettige Kohlenwasserstoffe mit 1 bis 8 C-Atomen, Schwefeltrioxid, Schwefeldioxid, Verunreinigungen aus Einsatzstoffen, z. B. aus dem eingesetzten Methan, Initiatoren und deren Zerfallsprodukte und Nebenprodukte der Reaktion beispielsweise Kohlenstoffmonoxid oder Kohlenstoffdioxid.

Als Schwersieder sind in der Rohalkansulfonsäure, insbesondere Methansulfonsäure, im Allgemeinen Schwefelsäure, Alkandisulfonsäure, insbesondere Methandisulfonsäure, langkettige Kohlenwasserstoffe, anorganische Salze wie Natriumsulfat, Natriumhydrogensulfat, Natriummethylsulfat, Ammoniumsulfit, Ammoniummethylsulfat, Calciumhydroxid, Calciumsulfat, Calciummethylsulfat enthalten. Wie die Leichtsieder sind dabei auch die in der Rohalkansulfonsäure enthaltenen Schwersieder abhängig vom Herstellprozess. So ist zum Beispiel bei den in der WO-A 00/31027, in der DE-C 197 43 901 und in der GB-A 1350328 beschriebenen Verfahren insbesondere Schwefelsäure als Schwersieder enthalten.

Bei einigen Herstellverfahren, zum Beispiel dem in der WO 2004/101860 beschriebenen, können zusätzlich auch Dialkylpolysulfide enthalten sein, die abhängig von ihrem Schwefelgehalt als Schwer- oder Leichtsieder auftreten. Demgegenüber enthält die nach dem Verfahren gemäß CN-A 1810780 hergestellte Rohalkansulfonsäure als Schwersieder Ammoniumsulfit, Ammoniumsulfat, Ammoniumsalze der Alkansulfonsäure, Calciumsalze der Alkansulfonsäure, Schwefelsäure, Calciumsulfat und Ammoniumhydrogensulfat.

Durch die Kristallisation können die in der Rohalkansulfonsäure enthaltenen Verunreinigungen entfernt werden. Diese reichern sich bei der Kristallisation in der Mutterlauge an. Dabei hängt es insbesondere vom Wassergehalt in der Ausgangsschmelze ab, ob Alkansulfonsäure oder das Hydrat der Alkansulfonsäure kristallisiert.

Chemische Verfahren, die Alkansulfonsäure wasserfrei herstellen, beispielsweise nach WO-A 2015/071365, enthalten kein Wasser als Leichtsieder. Dementsprechend ist eine Gewinnung der reinen Methansulfonsäure durch Kristallisation und nach Zusatz von Wasser eine Gewinnung von Methansulfonsäurehydrat durch Kristallisation möglich.

Die vollständige oder teilweise Entfernung der Leichtsieder durch die Destillation in Schritt (a) hat den weiteren Vorteil, dass hierdurch der Schmelzpunkt der Ausgangsschmelze aus Rohalkansulfonsäure für die nachfolgende Schmelzekristallisation erhöht wird. Hierdurch braucht die Ausgangsschmelze weniger stark abgekühlt zu werden, wodurch Energie zur Kühlung eingespart werden kann.

Da bei einer Kristallisation und einer nachfolgenden Fest-Flüssig-Trennung in der Regel keine vollständige Abtrennung des Produkts aus der Ausgangsschmelze möglich ist, enthält die den Kristallisator verlassende Mutterlauge noch einen großen Anteil an Produkt. Aus diesem Grund ist es bevorzugt, die an Alkansulfonsäure abgereicherte und den Kristallisator verlassende Mutterlauge vollständig oder zumindest teilweise wieder in den Prozess zur Aufreinigung von Alkansulfonsäuren zurückzuführen. Hierbei kann die an Alkansulfonsäure abgereicherte Mutterlauge entweder zurück in den Kristallisator geleitet werden, Kristallisationskreis genannt, oder alternativ vollständig oder teilweise zurück in die Destillation, Destillationskreis genannt. Die Rückführung in die Destillation hat dabei den Vorteil, dass Leichtsieder, die sich aufgrund der Entfernung des Produkts in der Mutterlauge anreichern, ebenfalls entfernt werden.

Bevorzugt ist, dass die gesamte Mutterlauge, die in Schritt (c) vom Kristallisat entfernt wird oder die aus dem Kristallisator abläuft, in die Destillation in Schritt (a) oder in die Schmelzekristallisation zurückgeführt wird.

Wenn zusätzlich das Waschen der Kristalle in Schritt (d) durchgeführt wird, fällt verunreinigte Waschflüssigkeit an, die in den Prozess zur Aufreinigung von Alkansulfonsäuren rückgeführt wird. In diesem Fall ist es bevorzugt, wenn die Waschflüssigkeit mit der Mutterlauge zusammengeführt wird.

Da sich in der Mutterlauge durch das Auskristallisieren der Alkansulfonsäure insbesondere bei Rückführung in die Destillation oder die Schmelzekristallisation Schwersieder anreichern, ist es weiterhin bevorzugt, wenn die in Schritt (c) entfernte Mutterlauge zumindest teilweise einer Schwersiederabtrennung zugeführt wird, um Schwersieder aus der Mutterlauge zu entfernen. Nach Abtrennung der Schwersieder wird die Mutterlauge in den Prozess zur Aufreinigung von Alkansulfonsäuren zurückgeführt. Die Zufuhr der Mutterlauge in die Schwersiederabtrennung und anschließende Rückführung in den Prozess zur Aufreinigung von Alkansulfonsäuren wird Schwersiederkreis genannt. Die Schwersiederabtrennung erfolgt dabei vorzugsweise vor der Einleitung der Mutterlauge in die Destillation in Schritt (a) oder in die Schmelzekristallisation. Besonders bevorzugt ist es dabei, wenn die Mutterlauge nach dem Entfernen der Schwersieder in die Destillation in Schritt (a) zurückgeführt wird. Dieses erlaubt es, dass noch in der Mutterlauge als Verunreinigungen enthaltene Leichtsieder in der Destillation ebenfalls aus der Mutterlauge entfernt werden. Alternativ ist es auch möglich, die Mutterlauge nach dem Entfernen der Schwersieder teilweise zu kondensieren und den kondensierten Anteil in die Destillation in Schritt (a) zurückzuführen und den nicht kondensierten Anteil als Leichtsieder aus dem Prozess auszuschleusen. Durch die Teilkondensation der Mutterlauge und das Ausschleusen des nicht kondensierten Anteils an Leichtsiedern wird bei einer Rückführung des kondensierten Anteils der Mutterlauge in die Destillation in Schritt (a) der in der Destillation zu entfernende Anteil an Leichtsiedern reduziert, so dass die Destillation insgesamt für einen geringeren Durchsatz auszulegen ist, wodurch wiederum Investitionskosten und insbesondere Energie eingespart werden können.

Alternativ ist es möglich, die Anreicherung von Schwersiedern in der Mutterlauge dadurch zu begrenzen, dass Teile der Mutterlauge ausgeschleust und beispielsweise als Abfallstrom behandelt und oder in irgendeiner Weise verwertet werden.

Die Schwersiederabtrennung kann beispielsweise durch eine Verdampfung realisiert werden. Hierbei verdampfen die in der Mutterlauge enthaltenen Leichtsieder und die Alkansulfonsäure und die Schwersieder verbleiben in der Flüssigphase. Um die Alkansulfonsäure nicht zu schädigen und die Verdampfung bei möglichst niedrigen Temperaturen durchführen zu können, wird die Verdampfung vorzugsweise bei einem Druck unterhalb des Umgebungsdrucks durchgeführt. Die bei der Verdampfung entstehenden, Alkansulfonsäure enthaltenden Brüden werden vorzugsweise kondensiert und als Flüssigkeit zurück in die Destillation in Schritt (a) oder in die Schmelzekristallisation in Schritt (b) geleitet. Durch die Kondensation der Brüden besteht auch die Möglichkeit, Leichtsieder zu entfernen. In diesem Fall wird die Kondensation so durchgeführt, dass die Alkansulfonsäure kondensiert, die Leichtsieder jedoch in der Gasphase verbleiben. In einem Gas-Flüssig-Phasentrenner lassen sich dann die die Alkansulfonsäure enthaltende Flüssigphase und die die Leichtsieder enthaltende Gasphase voneinander trennen und die in der Gasphase enthaltenen Leichtsieder können aus dem Prozess ausgeschleust werden. Eine Verdampfung kann auch im Sinne einer Strippung durch ein Schleppgas gefördert werden.

Die bei der Verdampfung anfallende Schwersieder-enthaltende Fraktion enthält im Allgemeinen auch noch einen Anteil an Alkansulfonsäure, die gegebenenfalls in einen nachfolgenden Reinigungsschritt zurückgewonnen werden kann.

Sowohl die Destillation in Schritt (a) als auch die Verdampfung der Alkansulfonsäure zur Entfernung von Schwersiedern werden bei einem Druck unterhalb des Umgebungsdrucks durchgeführt. Bevorzugt werden die Destillation in Schritt (a) und die Verdampfung zur Entfernung von Schwersiedern bei einem Druck im Bereich 5 bis 500 mbar (abs), besonders bevorzugt von 10 bis 100 mbar (abs) durchgeführt. Dies erlaubt eine für die Alkansulfonsäure produktschonende Destillation beziehungsweise Verdampfung. Bei höheren Drücken wären die notwendigen Temperaturen für die Destillation beziehungsweise Verdampfung so hoch, dass eine Produktschädigung, insbesondere eine Zersetzung der Alkansulfonsäure nicht ausgeschlossen werden kann. Es ist bekannt, dass Destillationsverfahren unter Einsatz von Schleppmitteln, sogenannten Strippverfahren, bei höheren Drücken durchgeführt werden können. Diese Prozessführung wird im Rahmen der Erfindung als äquivalent zur Anwendung eines Unterdrucks angesehen.

Wenn die Alkansulfonsäure Methansulfonsäure ist, die durch ein wasserfreies Verfahren hergestellt wird, ist in der Ausgangsschmelze kein Wasser sondern Schwefeltrioxid als Leichtsieder enthalten. Um reine Methansulfonsäure zu kristallisieren, ist die Leichtsiederabtrennung so zu führen, dass die Ausgangsschmelze eine Konzentration an Methansulfonsäure von mehr als 87 mol-%, bevorzugt mehr als 92 mol-% und besonders bevorzugt 93 bis 98 mol-% aufweist.

Wenn die Alkansulfonsäure Methansulfonsäure ist, die durch ein nicht wasserfreies Verfahren hergestellt wird und reine Methansulfonsäure aus der Methansulfonsäure und Wasser enthaltenden Ausgangsschmelze kristallisiert werden soll, ist die Leichtsiederabtrennung so durchzuführen, dass die Ausgangsschmelze eine Konzentration an Methansulfonsäure von mindestens 76 mol-%, bevorzugt mindestens 82 mol-% und besonders bevorzugt mindestens 90 mol-% aufweist. Die Druck- und Temperatureinstellungen der Leichtsiederabtrennung können dabei in weiten Bereichen variiert werden, sind aber durch die stoffspezifischen Dampfdruckkurven miteinander verbunden. Der besonders bevorzugte Wert der Methansulfonsäurekonzentration in einem Methansulfonsäure-Wassergemisch von 90 Mol.-% ist in einem bevorzugten Druckbereich von 40 bis 130 mbar (abs) und den korrespondierenden Sumpftemperaturbereichen von 160°C bis 200°C zu erreichen. Der angegebene Temperaturbereich ist um typischerweise 10 bis 20K höher als physikalisch stoffspezifisch vorgegeben, da durch Strömung in der Kolonne Druckverluste und durch Isolationsschwächen Wärmeverluste auftreten können.

Wenn die Alkansulfonsäure Methansulfonsäure ist und das Methansulfonsäurehydrat kristallisiert werden soll, ist die Leichtsiederabtrennung so durchzuführen, dass die Methansulfonsäure und Wasser enthaltende Ausgangsschmelze eine Konzentration an Methansulfonsäure von 31 bis 75 mol-%, bevorzugt von 45 bis 63 mol-% und besonders bevorzugt von 47 bis 55 mol-% aufweist. Die Druck- und Temperatureinstellungen der Leichtsiederabtrennung können auch hier in weiten Bereichen variiert werden. Ein besonders bevorzugter Wert der Methansulfonsäurekonzentration von 51 mol-% ist beispielsweise bei einem Druck von 40 bis 130 mbar (abs) und einer Sumpftemperatur von 80 bis 120°C zu erreichen.

Wenn die Alkansulfonsäure Ethansulfonsäure ist, die durch ein nicht wasserfreies Verfahren hergestellt wird und reine Ethansulfonsäure kristallisiert werden soll, ist die Leichtsiederabtrennung so durchzuführen, dass die Ethansulfonsäure und Wasser enthaltende Ausgangsschmelze eine Konzentration an Ethansulfonsäure von mindestens 76 mol-%, bevorzugt mindestens 82 mol-% und besonders bevorzugt mindestens 90 mol-% aufweist. Die Druck- und Temperatureinstellungen der Leichtsiederabtrennung können dabei in wie bei der Herstellung von Methansulfonsäure in weiten Bereichen variiert werden. Da Ethansulfonsäure einen deutlich niedrigeren Dampfdruck als Methansulfonsäure besitzt, liegen die bevorzugten Destillationsdrücke entsprechend tiefer. Eine alternative Erhöhung der Destillationstemperaturen weit über 200°C hinaus ist aufgrund merkbarer Ethansulfonsäure-Zersetzung schwierig.

Wenn die Alkansulfonsäure Ethansulfonsäure ist und das Ethansulfonsäurehydrat kristallisiert werden soll, ist die Leichtsiederabtrennung so durchzuführen, dass die Wasser und Ethansulfonsäure enthaltende Ausgangsschmelze eine Konzentration an Ethansulfonsäure von 31 bis 75 mol-%, bevorzugt von 45 bis 63 mol-% und besonders bevorzugt von 47 bis 55 mol-% aufweist.

Die Destillation kann dabei in jeder beliebigen, dem Fachmann bekannten Destillationseinrichtung durchgeführt werden. Üblicherweise wird die Destillation in einer Destillationskolonne durchgeführt, die Einbauten enthalten kann. Übliche Einbauten sind zum Beispiel Böden oder strukturierte oder unstrukturierte Packungen. Als Böden können alle bekannten Böden, zum Beispiel Siebböden, Glockenböden, Tunnelböden oder Ventilböden, eingesetzt werden. Strukturierte Packungen können beispielsweise solche aus Keramik oder Kunststoffen wie PFTE oder PFA sein. Unstrukturierte Packungen sind zum Beispiel Füllkörperpackungen, wobei alle gängigen Füllkörper verwendet werden können, z.B. solche aus Keramik, Kunstoffen wie PFTE oder PFA.

Im Allgemeinen wird die aus der Herstellung kommende Rohalkansulfonsäure nahe am Kopf der Destillationskolonne aufgegeben. Über Kopf werden die Leichtsieder abgetrennt und einer Aufarbeitung oder einer Entsorgung zugeführt. Am Sumpf der Destillationskolonne wird ein Alkansulfonsäure, Schwersieder und Reste an Leichtsiedern, insbesondere Wasser bei nicht wasserfrei arbeitenden Verfahren oder Schwefeltrioxid bei wasserfrei arbeitenden Verfahren, enthaltender Stoffstrom entnommen und der Schmelzekristallisation als Ausgangsschmelze zugeführt. Hierbei ist die Ausgangsschmelze im Allgemeinen einphasig flüssig. Das heißt, dass auch die Alkansulfonsäure vollständig in der flüssigen Phase enthalten ist.

Alternativ können jedoch auch alle anderen, dem Fachmann bekannten Verdampfungsapparate zur Durchführung der Destillation eingesetzt werden.

Da die Destillation und die Schmelzekristallisation bei unterschiedlichen Temperaturen durchgeführt werden, ist es unabhängig von der eingesetzten Destillationseinrichtung notwendig, den Alkansulfonsäure, Schwersieder und Reste an Leichtsiedern enthaltenden Stoffstrom abzukühlen, bevor dieser der Schmelzekristallisation zugeführt wird. Selbst bei beispielhaft einem Unterdruck von 100 mbar (abs) der Leichtsiederabtrennung ist es notwendig, die Destillation bei Wärmezufuhr durchzuführen, um die Sumpftemperatur im Fall der Kristallisation reiner Methansulfonsäure im Bereich von 160 bis 191°C und im Falle der Kristallisation von Methansulfonsäurehydrat im Bereich von 86 bis 112°C einzustellen. Da die Schmelztemperatur eines Methansulfonsäure-Wassergemisches abhängig vom Wassergehalt im Bereich von -54 bis +20°C liegt, muss zunächst eine entsprechende Abkühlung des Sumpfaustrages bevorzugt bis auf eine Temperatur knapp oberhalb der Schmelztemperatur der Ausgangsschmelze erfolgen. Alternativ ist es auch möglich, die Schmelze vor dem Eintritt in den Kristallisator zu unterkühlen. Eine solche Fahrweise ist allerdings nicht bevorzugt, da eine ungewollte Kristallisation in einem Wärmetauscher schwer auszuschließen ist. Wenn eine andere Alkansulfonsäure aufgereinigt werden soll, müssen die Temperaturen entsprechend an die Siedetemperatur beziehungsweise Schmelztemperatur der Alkansulfonsäure angepasst werden.

Durch die Destillation in Schritt (a) wird die Rohalkansulfonsäure vorzugsweise so weit von Leichtsiedern befreit, dass der Anteil an Verunreinigungen in dem der Schmelzekristallisation als Ausgangsschmelze zugeführten Alkansulfonsäure, Schwersieder und Reste an Leichtsiedern enthaltenden Stoffstrom ohne Berücksichtigung von enthaltenem Wasser bei nicht wasserfrei arbeitenden Verfahren beziehungsweise ohne Berücksichtigung von Schwefeltrioxid bei wasserfrei arbeitenden Verfahren maximal 6 Gew.-%, bevorzugt maximal 3 Gew.-% in der Ausgangsschmelze beträgt. Besonders bevorzugt ist es, wenn der Anteil an Verunreinigungen ohne Berücksichtigung von Wasser beziehungsweise Schwefeltrioxid kleiner als 2 Gew.-% ist. Es handelt sich bei den Angaben um nur typische Werte, die darüber hinaus abhängig vom Herstellverfahren der Alkansulfonsäure und dem Schwersiedergehalt sind.

Im Unterschied zur Destillation, die bei einem Druck unterhalb des Umgebungsdrucks durchgeführt wird, erfolgt die Schmelzekristallisation im Allgemeinen bei Umgebungsdruck. Im Falle der reinen Methansulfonsäure wird die Schmelzekristallisation dabei vorzugsweise bei einer Temperatur im Bereich von -10°C bis 19°C durchgeführt.

Für die Gewinnung von reiner Methansulfonsäure oder des Monohydrats der Methansulfonsäure in der Schmelzekristallisation ist insbesondere das Verhältnis von Wasser zu Methansulfonsäure maßgeblich. Nachfolgend werden als Verunreinigungen die Summe aller Substanzen ohne Wasser und Methansulfonsäure bezeichnet.

Um in der Schmelzekristallisation reine Methansulfonsäure zu gewinnen, das heißt, Methansulfonsäure mit einem Anteil an Verunreinigungen und Wasser von weniger als 1 Gew.-%, bevorzugt von weniger als 0,5 Gew.-% und insbesondere von weniger als 0,2 Gew.-%, wird der Schmelzekristallisation ein Alkansulfonsäure, Schwersieder und Reste an Leichtsiedern enthaltender Stoffstrom zugeführt, der mindestens 76 Mol.-%, bevorzugt mindestens 82 Mol.-% und insbesondere bevorzugt mindestens 90 Mol.-% Methansulfonsäure bezogen auf die Gesamtmenge an Methansulfonsäure und Wasser im Alkansulfonsäure, Schwersieder und Reste an Leichtsiedern enthaltenden Stoffstrom enthält.

Weiterhin enthält der Alkansulfonsäure, Schwersieder und Reste an Leichtsiedern enthaltende Stoffstrom vor der Einspeisung von Rückführungen maximal 6 Gew.-% Verunreinigungen, vorzugsweise maximal 3 Gew.-% und insbesondere maximal 2 Gew.-% bezogen auf die Gesamtmasse des Alkansulfonsäure, Schwersieder und Reste an Leichtsiedern enthaltenden Stoffstroms. Liegen im Stoffgemisch neben Wasser und Alkansulfonsäure Verunreinigungen vor, ändern sich die molaren zur Kristallisation geeigneten Konzentrationsverhältnisse zwischen Wasser und Alkansulfonsäure nicht wesentlich. Durch Rückführung im Schwersiederkreis können die Konzentrationen an Verunreinigungen, beispielsweise an Schwefelsäure, über die angegebenen Werte hinaus steigen und dabei den Anteil an Wasser verringern. Liegen höhere Konzentrationen an Verunreinigungen vor, sind tiefere Kristallisationstemperaturen möglich und erforderlich.

Zur Gewinnung der reinen Methansulfonsäure wird die Schmelzekristallisation bei einer Temperatur im Bereich von -50 bis 19°C und bevorzugt im Bereich von -10 bis 19°C, weiter bevorzugt bei einer Temperatur im Bereich von -2 bis 18°C und insbesondere bei einer Temperatur im Bereich von 6 bis 16°C durchgeführt. Hohe Kristallisationstemperaturen sind bevorzugt, da hierdurch der Energieaufwand bei der Kristallisation geringer ist als bei niedrigeren Kristallisationstemperaturen.

Zur Gewinnung des Monohydrats der Methansulfonsäure in der Schmelzekristallisation enthält der der Schmelzekristallisation als Ausgangsschmelze zugeführte Alkansulfonsäure, Schwersieder und Reste an Leichtsiedern enthaltende Stoffstrom vorzugsweise 31 bis 75 Mol.-% Methansulfonsäure, bevorzugt 45 bis 63 Mol.-%, und besonders bevorzugt 47 bis 55 Mol-% und insbesondere 48 bis 52 Mol.-% Methansulfonsäure jeweils bezogen auf die Gesamtmenge an Wasser und Methansulfonsäure im Alkansulfonsäure, Schwersieder und Reste an Leichtsiedern enthaltenden Stoffstrom. Auch hier liegt der Anteil an Verunreinigungen vorzugsweise bei maximal 6 Gew.-%, weiter bevorzugt bei maximal 3 Gew.-% und insbesondere bei maximal 2 Gew.-% bezogen auf die Gesamtmasse des Alkansulfonsäure, Schwersieder und Reste an Leichtsiedern enthaltenden Stoffstroms. Liegen in Stoffgemisch neben Wasser und Alkansulfonsäure Verunreinigungen vor, ändern sich die molaren zur Kristallisation geeigneten Konzentrationsverhältnisse zwischen Wasser und Alkansulfonsäure nicht wesentlich. Es handelt sich bei den Angaben um nur typische Werte, die darüber hinaus abhängig vom Methansulfonsäure-Herstellverfahren und dem Schwersiedergehalt sind. Liegen hohe Konzentrationen an Verunreinigungen vor, sind tiefere Kristallisationstemperaturen möglich und erforderlich.

Die Temperatur, bei der die Schmelzekristallisation des Monohydrats der Methansulfonsäure durchgeführt wird, liegt dabei im Bereich von -50 bis 12°C und bevorzugt im Bereich von -15 bis 12°C, weiter bevorzugt im Bereich von -8 bis 12°C und insbesondere im Bereich von 0 bis 12°C. Hohe Kristallisationstemperaturen sind bevorzugt, da sie einen niedrigeren Energieaufwand bei der Kristallisation benötigen.

Bei wasserfrei arbeitenden Verfahren ist für die Gewinnung reiner Methansulfonsäure das Verhältnis von Schwefeltrioxid zu Methansulfonsäure maßgeblich. In diesem Fall werden als Verunreinigungen die Summe aller Substanzen ohne Schwefeltrioxid und Methansulfonsäure bezeichnet.

Um in der Schmelzekristallisation reine Methansulfonsäure zu gewinnen, das heißt, Methansulfonsäure mit einem Anteil an Verunreinigungen und Schwefeltrioxid von weniger als 1 Gew.-%, bevorzugt von weniger als 0,5 Gew.-% und insbesondere von weniger als 0,2 Gew.-%, wird der Schmelzekristallisation ein Alkansulfonsäure, Schwersieder und Reste an Leichtsiedern enthaltender Stoffstrom zugeführt, der mindestens 87 mol-%, bevorzugt mindestens 92 mol-% und insbesondere bevorzugt 93 bis 98 mol-% Methansulfonsäure bezogen auf die Gesamtmenge an Methansulfonsäure und Schwefeltrioxid im Alkansulfonsäure, Schwersieder und Reste an Leichtsiedern enthaltenden Stoffstrom enthält.

Weiterhin enthält der Alkansulfonsäure, Schwersieder und Reste an Leichtsiedern enthaltende Stoffstrom vor der Einspeisung von Rückführungen maximal 6 Gew.-% Verunreinigungen, vorzugsweise maximal 3 Gew.-% und insbesondere maximal 2 Gew.-% bezogen auf die Gesamtmasse des Alkansulfonsäure, Schwersieder und Reste an Leichtsiedern enthaltenden Stoffstroms. Liegen im Stoffgemisch neben Schwefeltrioxid und Alkansulfonsäure Verunreinigungen vor, ändern sich die molaren zur Kristallisation geeigneten Konzentrationsverhältnisse zwischen Schwefeltrioxid und Alkansulfonsäure nicht wesentlich. Durch Rückführung im Schwersiederkreis können die Konzentrationen an Verunreinigungen, beispielsweise an Schwefelsäure, über die angegebenen Werte hinaus steigen und dabei den Anteil an Schwefeltrioxid verringern. Liegen höhere Konzentrationen an Verunreinigungen vor, sind tiefere Kristallisationstemperaturen möglich und erforderlich.

Zur Gewinnung der reinen Methansulfonsäure wird die Schmelzekristallisation bei einer Temperatur im Bereich von -50 bis 19°C und bevorzugt im Bereich von -10 bis 19°C, weiter bevorzugt bei einer Temperatur im Bereich von -2 bis 18°C und insbesondere bei einer Temperatur im Bereich von 6 bis 16°C durchgeführt. Hohe Kristallisationstemperaturen sind bevorzugt, da hierdurch der Energieaufwand bei der Kristallisation geringer ist als bei niedrigeren Kristallisationstemperaturen.

Je nach Art und Konzentration der Verunreinigungen können die optimalen Kristallisationsbedingungen unterschiedlich sein. Diese sollten entsprechend zum Beispiel durch Versuche angepasst werden. Bei kleinen Konzentrationen an Verunreinigungen liegen die Kristallisationsbedingungen sehr nahe an denen des reinen Zweistoffgemischs aus Wasser beziehungsweise Schwefeltrioxid und Methansulfonsäure.

Es hat sich gezeigt, dass sowohl bei der Herstellung der reinen Methansulfonsäure als auch bei der Herstellung des Hydrats der Methansulfonsäure jeweils ein Anteil von 6 Gew.-% bezogen auf die Gesamtmasse des Alkansulfonsäure, Schwersieder und Reste an Leichtsiedern enthaltenden Stoffstromes Schwefelsäure unkritisch ist und die Kristallisationsbedingungen nur gering beeinflusst. Beispielsweise zeigt sich, dass eine Verunreinigung von 4 Gew.-% Schwefelsäure in einem Gemisch aus Wasser und Methansulfonsäure die Kristallisationstemperatur von Methansulfonsäure um ca. 3°C absenkt. Beispielsweise zeigt sich auch, dass eine Verunreinigung von 4 Gew.-% Schwefelsäure in einem Gemisch aus Wasser und Methansulfonsäure die Kristallisationstemperatur von Methansulfonsäurehydrat um ca. 2°C absenkt.

Als Kristallisator, in dem die Schmelzekristallisation durchgeführt wird, kann jeder für eine Kristallisation geeignete Apparat eingesetzt werden. Die Wärme kann im Kristallisator zum Beispiel durch Mantelkühlung oder durch geeignete Einbauten, beispielsweise von einem Kältemittel durchströmte Rohrleitungen im Kristallisator entzogen werden, bis eine für die Kristallisation ausreichend niedrige Temperatur erreicht ist. Als Kältemittel, das bei der Mantelkühlung durch eine Doppelwandung des Kristallisators strömt oder das in der durchströmten Rohrleitung eingesetzt wird, eignet sich zum Beispiel ein Gemisch aus Wasser und Ethylenglykol. Alternativ ist es auch möglich, eine direkte Kühlung durch ein verdampfendes Kältemittel, beispielsweise Kohlenstoffdioxid, durchzuführen.

Im Kristallisator wird die Ausgangschmelze in einer Ausführungsform, dem Suspensionskristallisationsverfahren, durch Abkühlung in eine Alkansulfonsäure-Kristalle enthaltende Suspension überführt. Hierzu können Feststoffkristalle aus der Alkansulfonsäure direkt in der Schmelze wachsen und so die Suspension bilden oder alternativ können sich die Feststoffkristalle als Schicht auf einer gekühlten Wand abscheiden, von der sie anschließend abgekratzt und in der Mutterlauge resuspendiert werden. Als Apparate eignen sich zum Beispiel Rührkessel, Kratzkühler oder Scheibenkristallisatoren.

In einer alternativen Ausführungsform wird eine Schichtkristallisation durchgeführt. Hierbei bildet sich das Kristallisat als zusammenhängende anhaftende Schicht an einer gekühlten Oberfläche des Kristallisators. Die Fest/Flüssig-Trennung erfolgt in diesem Fall durch Abfließen der Mutterlauge in einem Schwerefeld. Die Schichtkristallisation kann sowohl als statische als auch als dynamische Schichtkristallisation durchgeführt werden.

Bei der statischen Schichtkristallisation wird die Ausgangsschmelze in einen geeigneten Wärmetauscher eingefüllt, beispielsweise einen Rohrbündelwärmetauscher oder einen Plattenwärmetauscher, und durch langsame Temperaturabsenkung abgekühlt, wodurch die Ausgangsschmelze teilweise erstarrt. In einem weiteren Schritt wird die Mutterlauge abgelassen und die Temperatur wieder erhöht. Hierdurch wird zunächst eine stark verunreinigte Fraktion aus der Kristallschicht abgeschmolzen, bevor das Produkt mit hoher Reinheit abgeschmolzen wird. Nachteil des statischen Kristallisationsverfahrens ist jedoch die üblicherweise geringe Raum-Zeit-Ausbeute, da der Wärme- und Stofftransport zu den Abscheideflächen nur durch freie Konvektion erfolgt. Im Unterschied dazu wird bei der dynamischen Schichtkristallisation eine erzwungene Konvektion durch Umpumpen der Mutterlauge durch volldurchströmte Rohre, durch Aufgabe als Rieselfilm oder durch Einleiten von Inertgas in ein mit Mutterlauge gefülltes Rohr oder durch Pulsieren erzeugt.

Bei der Suspensionskristallisation wird dem Kristallisator eine Suspension entnommen, bei der die Kristalle in der Mutterlauge suspendiert sind. Da aus der Ausgangsschmelze Alkansulfonsäure kristallisiert ist, ist der Anteil der geschmolzenen Alkansulfonsäure in der dem Kristallisator entnommenen Mutterlauge geringer als in der dem Kristallisator zugeführten Ausgangsschmelze. Auch ist die Konzentration an Verunreinigungen in der Mutterlauge höher, da diese weitgehend nicht kristallisieren. Als Mutterlauge wird nur der flüssige Anteil, das heißt die flüssige Phase der Suspension, bezeichnet.

Um die Mutterlauge und an den Kristallen anhaftende Verunreinigungen zu entfernen, ist es möglich und bevorzugt, die Kristalle in Schritt (d) zu waschen. Hierzu werden die Kristalle mit einer Waschflüssigkeit in Kontakt gebracht, mit der die Verunreinigungen entfernt werden.

Zum Waschen der Kristalle im Schritt (d) kann jeder geeignete Wäscher genutzt werden. Hierbei ist es möglich, einen separaten Wäscher einzusetzen oder die Fest-Flüssig-Trennung und Waschung in einem Apparat durchzuführen. Ein dazu geeigneter Apparat ist zum Beispiel eine Waschkolonne. In der Waschkolonne werden die zu reinigenden Kristalle und die Waschflüssigkeit vorzugsweise im Gegenstrom geführt. Da Alkansulfonsäuren, insbesondere Methansulfonsäure, korrosiv sind, ist es notwendig, neben den Produktionsapparaten auch den Kristallisator, den Apparat zur Fest-Flüssig-Trennung und den Wäscher so auszustatten, dass dieser verfahrenstechnisch beständig ist. Insbesondere muss vermieden werden, dass die Alkansulfonsäure durch korrodierte und sich ablösende Bestandteile des Apparats verschmutzt wird. Geeignete korrosionsstabile Materialien, aus denen der Wäscher gefertigt werden kann, sind zum Beispiel Gläser, korrosionsfeste Stähle, emaillierte Stähle oder Kunststoffe. Die Kunststoffe können dabei sowohl als Auskleidungsmaterialien als auch tragend zum Einsatz kommen. Ein geeigneter Kunststoff ist zum Beispiel Polyethylen hoher Dichte oder auch PTFE. Kunststoffe sind vor allem als Konstruktionsmaterial oder zur korrosiven Isolierung des Apparateaußenmantels geeignet. Einige Apparateteile können möglicherweise für Kunststoffe zu hoch mechanisch belastet sein. Dann kann eine Konstruktion so erfolgen, dass die belasteten Anlagenteile beispielsweise auch aus mechanisch stabilem, emailliertem Stahl gefertigt werden.

Als Waschflüssigkeit können zum Beispiel Wasser, wässrige Alkansulfonsäure, Schwefelsäure oder andere Lösemittel eingesetzt werden. Diese haben jedoch alle den Nachteil, dass die Kristalle aus der Alkansulfonsäure gelöst werden können. Zudem können zusätzlich Verunreinigungen eingebracht werden. Anstelle der vorstehend genannten Waschflüssigkeiten ist es daher bevorzugt, geschmolzenes Kristallisat als Waschlösung einzusetzen. Mit dem geschmolzenen Kristallisat werden die an den Kristallen anhaftende Mutterlauge und die Verunreinigungen entfernt. Da das als Waschflüssigkeit eingesetzte geschmolzene Kristallisat durch die Mutterlauge und durch die Verunreinigungen, die von den Kristallen abgewaschen werden, verunreinigt wird und die Waschflüssigkeit aufgrund ihrer Zusammensetzung einen hohen Anteil an Wertprodukt enthält, ist es bevorzugt, das als Waschflüssigkeit eingesetzte geschmolzene Kristallisat nach dem Waschen der Kristalle in die Destillation in Schritt (a) oder in die Schmelzekristallisation zurückzuführen.

Wenn geschmolzenes Kristallisat als Waschflüssigkeit genutzt wird, kristallisiert im Allgemeinen auch ein Teil der Waschflüssigkeit an den zu reinigenden Kristallen aus.

Um ein Sedimentieren der Kristalle aus der Suspension beim Transport zwischen den einzelnen Apparaten, insbesondere zwischen dem Kristallisator und dem Wäscher zu verhindern, ist es bevorzugt, die Suspension zu homogenisieren. Hierzu können zum Beispiel Rührer oder Pumpen eingesetzt werden. Dem Wäscher kann entweder direkt die dem Kristallisator entnommene Suspension zugeführt werden oder alternativ ist es auch möglich, die Suspension vor der Zugabe in den Wäscher aufzubereiten. Hierzu werden die in der Mutterlauge suspendierten Kristalle zunächst mechanisch abgetrennt. Hierzu kann jedes bekannte Trennverfahren für Fest/Flüssig-Trennungen eingesetzt werden. Geeignete Trennverfahren sind zum Beispiel Sieben, Pressen, Filtration, Zentrifugation, Sedimentation und Dekantieren. Nach dem Abtrennen der Mutterlauge werden die Kristalle in der Waschflüssigkeit resuspendiert und die Suspension in den Wäscher eingeleitet.

Wenn geschmolzenes Kristallisat als Waschflüssigkeit eingesetzt wird, ist es bevorzugt, wenn die Temperatur so gewählt wird, dass das geschmolzene Kristallisat zum Waschen der Kristalle eine Temperatur aufweist, die 0,1 bis 15°C oberhalb der Erstarrungstemperatur der Alkansulfonsäure-haltigen Kristallisats liegt. Bevorzugt liegt die Temperatur als Waschflüssigkeit eingesetzten Kristallisats 1 bis 10°C oberhalb der Erstarrungstemperatur der Alkansulfonsäure und insbesondere 2 bis 4°C oberhalb der Erstarrungstemperatur der Alkansulfonsäure.

Der Wäscher wird dabei vorzugsweise so betrieben, dass die Verweilzeit der zu waschenden Kristalle in dem Wäscher im Bereich von 1 bis 25 min und bevorzugt im Bereich von 1 bis 15 min liegt. Insbesondere, aber nicht ausschließlich bei Gegenstromführung von Kristalle enthaltender Suspension und geschmolzenem Kristallisat als Waschflüssigkeit hat sich allerdings gezeigt, dass bereits mit einer Verweilzeit von 2 bis 8 min eine ausreichende Reinigungswirkung erzielt wird.

Um die Reinigungswirkung zu verbessern, ist es möglich, die Kristalle mehrfach zu waschen. Hierzu kann das Waschen in Schritt (d) oder auch die Sequenz aus Kristallisation in Schritt (b), die Fest-Flüssig-Trennung in Schritt (c) und das Waschen in Schritt (d) mehrfach durchlaufen werden oder auch mit einer Teilrückführung betrieben werden. Bevorzugt ist jedoch ein einmaliger Durchlauf durch Kristallisation und Waschung. Bei geringen Ansprüchen an die Produktreinheit kann auch auf das Waschen der Kristalle verzichtet werden.

Die drei genannten Stoffstromkreise Destillationskreis, Kristallisationskreis und Schwersiederkreis führen über Anlagenbereiche, die auf teilweise sehr unterschiedlichen Temperaturniveaus liegen. Um die in das Verfahren eingebrachte Energie gut zu nutzen und weiterhin die Energiemenge, die zum Erwärmen und Abkühlen der Stoffströme benötigt wird, möglichst gering zu halten, ist es bevorzugt, wenn die Stoffstromkreise über Wärmeübertrager geführt werden, die im Gegenstrom Wärme übertragen. Beispielsweise wird der aus dem Sumpfaustrag der Leichtsiederdestillation entnommene, Alkansulfonsäure, Schwersieder und Reste an Leichtsiedern enthaltende Stoffstrom vor Zugabe in die Schmelzekristallisation abgekühlt und im Gegenzug die an Alkansulfonsäure abgereicherte Mutterlauge, die in die Destillation zurückgeführt wird, aufgeheizt. Dabei ist es besonders bevorzugt, wenn Wärme von dem abzukühlenden, Alkansulfonsäure, Schwersieder und Reste an Leichtsiedern enthaltenden Stoffstrom an die zu heizende und an Alkansulfonsäure abgereicherte Mutterlauge übertragen wird.

Wenn effiziente Kristallisations- und Waschverfahren zur Verfügung stehen, ist es möglich, im Schwersiederkreis, das heißt der im Kreis gefahrenen Mutterlauge, die Leichtsieder und Schwersieder zu hohen Konzentrationen aufzupegeln, ohne dass die kristallisierten Alkansulfonsäuren marktgerechte Reinheitsspezifikationen verfehlen. Hierbei hat sich in Versuchen gezeigt, dass Verunreinigungen an Leichtsiedern und Schwersiedern ohne Wasser beziehungsweise Schwefeltrioxid in Summe von mindestens bis zu 6 Gew.-% bezogen auf die Gesamtmasse des Stoffstromes unschädlich toleriert werden können. Hierdurch können die über hohe Temperaturdifferenzen geführten Strommengen im Schwersiederkreis so klein gehalten werden, dass deren Erwärmungs- und Abkühlaufwand klein ist gegenüber dem ohnehin verfahrenstechnisch notwendigen Wärmebedarf. Das erfindungsgemäße Verfahren benötigt somit weniger Energie als die herkömmlichen Verfahren.

Ausführungsbeispiele der Erfindung sind in den Figuren dargestellt und werden in der nachfolgenden Beschreibung näher erläutert.

Es zeigen:
- Figur 1: eine Übersichtsdarstellung eines Verfahren zur Alkansulfonsäureherstellung,
- Figur 2: ein Verfahrensfließbild des erfindungsgemäßen Verfahrens.

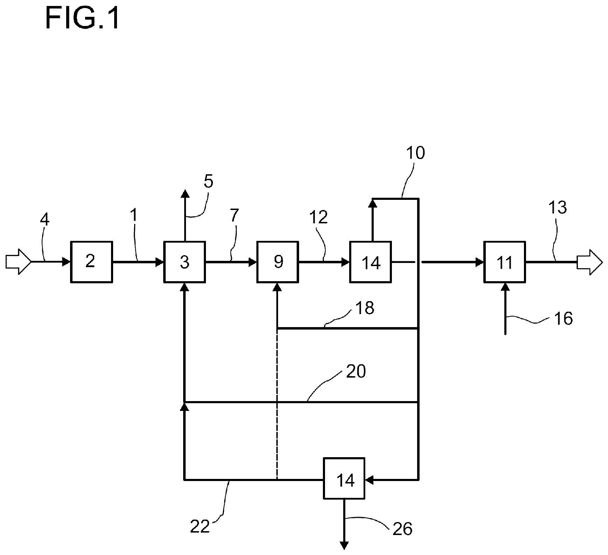

Ein Übersichtsdiagramm eines Verfahrens zur Alkansulfonsäureherstellung ist in Figur 1 dargestellt.

Einem ersten Prozessschritt 2 werden Einsatzstoffe 4 zur Herstellung von Alkansulfonsäure zugeführt. Die Einsatzstoffe 4 sind dabei abhängig vom Herstellprozess. Im ersten Prozessschritt 2 wird Rohalkansulfonsäure 1 hergestellt. An den ersten Prozessschritt 2 schließt sich eine Leichtsiederabtrennung 3 an, in der Leichtsieder aus der Rohalkansulfonsäure abgetrennt werden. Der Leichtsiederabtrennung 3 wird ein Leichtsieder enthaltender Stoffstrom 5 und ein Alkansulfonsäure, Schwersieder und Reste an Leichtsiedern enthaltender Stoffstrom 7 entnommen. In der Leichtsiederabtrennung 3 wird dabei eine zur Kristallisation geeignete Alkansulfonsäure-Konzentration in dem Alkansulfonsäure, Schwersieder und Reste an Leichtsiedern enthaltenden Stoffstrom 7 eingestellt.

Der Alkansulfonsäure, Schwersieder und Reste an Leichtsiedern enthaltende Stoffstrom 7 wird einer Schmelzekristallisation 9 als Ausgangsschmelze zugeführt. In der Schmelzekristallisation 9 wird die Ausgangsschmelze teilkristallisiert und eine Suspension 12 aus Mutterlauge und einer kristallinen Festphase aus Alkansulfonsäure erzeugt, die einer Fest-Flüssig-Trennung 14 zugeführt wird. In der Fest-Flüssig-Trennung 14 wird die Mutterlauge 10 weitgehend von der kristallinen Festphase getrennt. Die Festphase wird einem Wäscher 11 zugeführt, in dem mit Hilfe einer Waschflüssigkeit 16 an den Kristallen anhaftende Mutterlaugereste entfernt werden und das Produkt 13 gewonnen wird. Die Waschflüssigkeit wird vorzugsweise in die Schmelzekristallisation oder die Leichtsiederabtrennung zurückgeführt.

Die in der Fest-Flüssig-Trennung 14 abgetrennte Mutterlauge 10 wird vorzugsweise in den Prozess zur Aufreinigung von Alkansulfonsäuren zurückgeführt. Mögliche geeignete Rückführungen für die Mutterlauge 10 sind beispielhaft dargestellt. So kann zum Beispiel in einem Kristallisationskreis 18 Mutterlauge 10 ganz oder teilweise in die Schmelzekristallisation 9 zurückgeführt werden. Alternativ oder zusätzlich ist es möglich, Mutterlauge 10 in einem Destillationskreis 20 ganz oder teilweise in die Leichtsiederabtrennung 3 zurückzuführen. Weiterhin ist es möglich, in einem Schwersiederkreis 22 in einer Schwersiederabtrennung 19 einen Schwersiederpurgestrom 26 abzutrennen und den so durch die Abtrennung der Schwersieder aufgearbeiteten Strom in die Leichtsiederabtrennung 3 oder die Schmelzekristallisation 9 zurückzuführen.

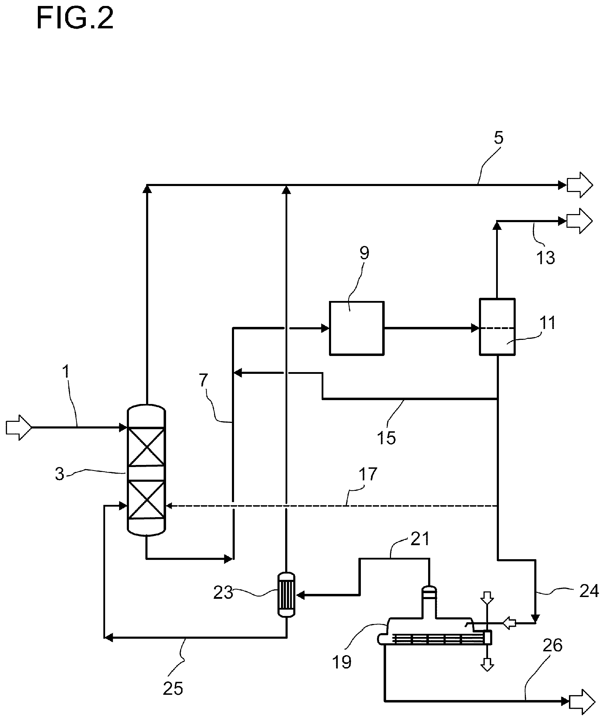

Figur 2 zeigt ein Verfahrensfließbild des erfindungsgemäßen Verfahrens.

Zur Aufreinigung wird eine Rohalkansulfonsäure enthaltende Schmelze 1, die einem Verfahren zur Herstellung von Alkansulfonsäure entstammt, in eine Leichtsiederabtrennung 3 eingeleitet. In der Leichtsiederabtrennung 3 werden Leichtsieder von der Rohalkansulfonsäure abgetrennt. Für die Leichtsiederabtrennung 3 kann zum Beispiel eine Destillationskolonne eingesetzt werden. Dabei fällt am Kopf der zur Leichtsiederabtrennung 3 eingesetzten Destillationskolonne ein Leichtsieder enthaltender Stoffstrom 5 an. Der Leichtsieder enthaltende Stoffstrom 5 wird aus dem Prozess entfernt und separat aufgearbeitet oder verworfen. Am Sumpf der zur Leichtsiederabtrennung 3 eingesetzten Destillationskolonne wird ein Alkansulfonsäure, Schwersieder und Reste an Leichtsiedern enthaltender Stoffstrom 7 entnommen. Dieser wird als Ausgangsschmelze einer Schmelzekristallisation 9 zugeführt. Die Schmelzekristallisation 9 kann, wie vorstehend beschrieben, zum Beispiel eine Suspensionskristallisation oder eine Schichtkristallisation sein, wobei die Schmelzekristallisation in jedem beliebigen, für das jeweilige Kristallisationsverfahren geeigneten Kristallisator durchgeführt werden kann.

Der Schmelzekristallisation 9 wird ein Stoffstrom entnommen, der Alkansulfonsäurekristalle und an Alkansulfonsäure abgereicherte Mutterlauge enthält. Dieser Stoffstrom wird einer Fest-Flüssig-Trennung und einem Wäscher 11 zugeführt, in dem die Alkansulfonsäurekristalle abgetrennt und zur Entfernung der daran anhaftenden Mutterlauge und Verunreinigungen gewaschen werden. Hierzu ist es zum Beispiel möglich, die in Mutterlauge suspendierten Alkansulfonsäurekristalle im Gegenstrom zu einer Waschflüssigkeit, beispielsweise geschmolzenem Kristallisat, zu führen. Die Waschflüssigkeit wäscht die Mutterlauge und Verunreinigungen von den Kristallen ab. Nach Trennung der Kristalle von der Waschflüssigkeit durch ein Fest/Flüssig-Trennverfahren wird reines Produkt 13 gewonnen, das aus dem Prozess entnommen wird. Wenn gewünscht, kann die gewonnene Alkansulfonsäure anschließend durch Zugabe von Wasser auf eine gewünschte Konzentration verdünnt werden.

Die Mutterlauge und Verunreinigungen enthaltende Waschflüssigkeit wird ebenfalls dem Apparat zur Fest-Flüssig-Trennung und Wäscher 11 entnommen. Da dieser Stoffstrom noch einen großen Anteil an Alkansulfonsäure enthält, wird dieser vorzugsweise nicht aus dem Prozess entfernt.

So ist es zum Beispiel möglich, den Waschflüssigkeit und an Alkansulfonsäure abgereicherten, dem Wäscher 11 entnommenen Stoffstrom als Rückführung 15 in die Schmelzekristallisation 9 zurückzuführen. Hierbei kann entweder der gesamte Strom oder nur ein Teilstrom zurückgeführt werden.

Um bei dieser Rückführung einer unzulässig hohen Aufpegelung von Leichtsiedern entgegen zu wirken, ist es möglich, die Waschflüssigkeit und den an Alkansulfonsäure abgereicherten, dem Wäscher entnommenen Strom als Rückführung 17 in die Leichtsiederabtrennung 3 zurückzuführen. Dies ist insbesondere dann sinnvoll, wenn in der Schmelzekristallisation 9 reine Alkansulfonsäure und nicht das Monohydrat der Alkansulfonsäure als Produkt gewonnen wird. Bei der Kristallisation des Monohydrates lässt sich dagegen eine geeignete Leichtsiederkonzentration alternativ durch Einstellung der Sumpftemperatur der zur Leichtsiederabtrennung 3 eingesetzten Destillationskolonne einstellen. Selbstverständlich ist es auch möglich, einen Teilstrom des Waschflüssigkeit und an Alkansulfonsäure abgereicherte Mutterlauge enthaltenden Stoffstroms als Rückführung 15 in die Schmelzekristallisation 9 zurückzuführen und einen weiteren Teilstrom in die Leichtsiederabtrennung 3.

Um Schwersieder aus dem Waschflüssigkeit und an Alkansulfonsäure abgereicherten, dem Wäscher entnommenen Stoffstrom zu entfernen, wird zumindest ein Teil der Mutterlauge, der so genannte Schwersiederpurge 24, einem Verdampfer 19 zugeführt oder anders genutzt. Im Verdampfer 19 werden Alkansulfonsäure und Leichtsieder verdampft und als Brüden 21 abgezogen. Die nicht verdampften Anteile werden als aufgearbeiteter Schwersiederpurgestrom 26 aus dem Verdampfer als Flüssigkeit entnommen und einer Weiternutzung oder Entsorgung zugeführt.

Die dem Verdampfer 19 entnommenen Brüden 21 können in den Prozess zur Aufreinigung von Alkansulfonsäuren, zurückgeführt oder einer anderen Nutzung, beispielsweise der Herstellung von MSA 100 Gew.-%, zugeführt werden. In der hier dargestellten Ausführungsform strömen die Brüden 21 in einen Teilkondensator 23. Im Teilkondensator 23 kondensiert die in den Brüden enthaltene Alkansulfonsäure und wird als Kondensat 25 in die Leichtsiederabtrennung 3 zurückgeführt. Die Brüden können ohne Teilkondensation nur aufwändig direkt gasförmig in die Leichtsiederabtrennung 3 geführt werden, da der Verdampfer 19 und die zur Leichtsiederabtrennung 3 eingesetzte Destillationskolonne üblicherweise auf unterschiedlichen Druckniveaus arbeiten. Eine alternative Rückführungsmöglichkeit der kondensierten Brüden besteht nach Abkühlung in der Nutzung als Waschflüssigkeit 16 für den Wäscher 11.

Der nicht kondensierte und Leichtsieder enthaltende Anteil wird dem Teilkondensator 23 gasförmig entnommen und zusammen mit dem Leichtsieder enthaltenden Stoffstrom 5 aus der Destillationskolonne 3 aus dem Prozess abgezogen.

Wenn nur ein Teil des dem Wäscher entnommenen und Waschflüssigkeit und abgereicherte Mutterlauge enthaltenden Stoffstroms in den Verdampfer 19 eingeleitet wird, kann der restliche Teil entweder als Rückführung 15 in die Schmelzekristallisation 9 oder als Rückführung 17 in die Destillationskolonne 3 oder auch teilweise als Rückführung 15 in die Schmelzekristallisation und teilweise als Rückführung 17 in die Destillationskolonne 3 zurückgeführt werden.

Aus Gründen der Energieeinsparung ist es sinnvoll, die Menge des Schwersiederpurges 24 so klein wie möglich zu wählen. Die Verringerung der Menge des Schwersiederpurges 24 wird dadurch nach unten begrenzt, dass noch ausreichende Mengen an Schwersieder aus dem Kreisprozess ausgeschleust werden, um deren Konzentration unterhalb der Konzentration zu halten, ab der sie die Kristallisation stören.

### Beispiele

### Kristallisation von Methansulfonsäure und Methansulfonsäurehydrat

Ausgangsschmelzen 1 bis 4 gemäß Tabelle 1 enthaltend Methansulfonsäure und Wasser sowie definierte Verunreinigungen, beispielsweise Schwefelsäure, Salpetersäure, Methansulfonsäuremethylester und Chlor (als Gesamtchlor), wurden bei Atmosphärendruck und Raumtemperatur einem Doppelmantelrührbehälter mit einem Fassungsvermögen von 1 l und einem Durchmesser von 150 mm mit einem wandgängigen Wendelrührer eingefüllt. Danach wurden die Ausgangsschmelzen mit einer Abkühlrate von 1 K/h auf die jeweils in Tabelle 1 angegebene Endtemperatur abgekühlt.

Bei der Abkühlung entstanden Kristalle, die durch Rühren mit einer Drehgeschwindigkeit von 180 min⁻¹ in Suspension gehalten wurden. Aus der Ausgangsschmelze 1 wurde reine Methansulfonsäure kristallisiert, aus den Ausgangsschmelzen 2 bis 4 wurde jeweils reines Methansulfonsäurehydrat kristallisiert. Die erhaltenen Kristalle in der Suspension wurden auf einem Druckfilter bei der jeweiligen Endtemperatur abgetrennt und mit einer 70%igen Methansulfonsäure-Wasser-Lösung gewaschen, um die an Kristallen anhaftende Mutterlauge teilweise zu entfernen. Dabei wurden die Kristallisatmenge und die Waschflüssigkeitsmenge gleich groß gewählt.

Der Wasseranteil in den gewaschenen Kristallen und in der Mutterlauge - außer in den Kristallen und in der Mutterlauge aus Ausgangsschmelze 4 - wurde mittels Karl-Fischer-Titration bestimmt. Die Anteile an Schwefelsäure und Salpetersäure in den gewaschenen Kristallen und in der Mutterlauge wurden mittels lonenchromatographie (IC) erfasst. Der Anteil an Methansulfonsäuremethylester in den gewaschenen Kristallen und in der Mutterlauge wurde mittels Gaschromatographie (GC) bestimmt. Der Anteil an Gesamtchlor in den gewaschenen Kristallen und in der Mutterlauge wurde durch Coulometrie ermittelt.

In Tabellen 2 und 3 sind die Anteile an Wasser und Verunreinigungen zusammengefasst. In Tabelle 4 werden die Verteilungskoeffizienten der Verunreinigungen dargestellt. Der Verteilungskoeffizient einer Komponente ist der Anteil der Komponente in den Kristallen geteilt durch Anteil der Komponente in der Mutterlauge. Verteilungskoeffizienten kleiner als 1 zeigen, dass die Anteile an Verunreinigungskomponente in den Kristallen kleiner sind als der Anteil in der Mutterlauge, das heißt, dass die Verunreinigung durch Kristallisation abgereichert wird.

Die in Tabelle 4 dargestellten Ergebnisse zeigen, dass ausgehend von den beispielhaften Ausgangsschmelzen 1 bis 4 Verunreinigungen aus Methansulfonsäure und Methansulfonsäurehydrat durch Kristallisation entfernt werden können.

Es lässt sich erkennen, dass abhängig von den Reinheitsanforderungen eine weitere Abreicherung der Verunreinigungen bei der Kristallisation gelingt, indem das Kristallisat nicht nur einfach sondern mehrfach kristallisiert und/oder im Gleichstrom oder im Gegenstrom gewaschen wird.

**Tabelle 1**

| Zusammensetzungen und Mengen der eingesetzten Ausgangsschmelzen 1-4 sowie die jeweils zugehörige Endtemperatur bei der Kristallisation | | | | |
|---|---|---|---|---|
| Ausgangsschmelze | 1 | 2 | 3 | 4 |
| Gesamtmenge [g] | 1250 | 1135 | 1390 | 1080 |
| Methansulfonsäure [Gew.-%] | 96,02 | 81,59 | 79,55 | 83,32 |
| Wasser [Gew.-%] | 1,96 | 15,51 | 15,21 | 15,68 |
| Schwefelsäure [Gew.-%] | 1,97 | 1,90 | 4,70 | - |
| Salpetersäure [Gew.-%] | - | 1 | 0,54 | - |
| Methansulfonsäuremethylester [ppm] | 500 | - | - | - |
| Gesamtchlor [Gew.-%] | - | - | - | nicht bestimmt |
| Endtemperatur [°C] | 4 | 6 | 4 | 4 |

**Tabelle 2**

| Wasser und Verunreinigungen in der Mutterlauge | | | | |
|---|---|---|---|---|
| Ausgangsschmelze | 1 | 2 | 3 | 4 |
| Wasser [Gew.-%] | 3,35 | 16,08 | 15,4 | nicht bestimmt |
| Schwefelsäure [Gew.-%] | 3,21 | 3,97 | 8,8 | - |
| Salpetersäure [Gew.-%] | - | 1,96 | 0,89 | - |
| Methansulfonsäuremethylester [ppm] | 1490 | - | - | - |
| Gesamtchlor [Gew.-%] | - | - | - | 0,5 |

**Tabelle 3**

| Wasser und Verunreinigungen in den gewaschenen Kristallen | | | | |
|---|---|---|---|---|
| Ausgangsschmelze | 1 | 2 | 3 | 4 |
| Wasser [Gew.-%] | 1,24 | 17,76 | 17,72 | nicht bestimmt |
| Schwefelsäure [Gew.-%] | 0,48 | 0,47 | 0,85 | - |
| Salpetersäure [Gew.-%] | - | 0,229 | 0,062 | - |
| Methansulfonsäuremethylester [ppm] | 185 | - | - | - |
| Gesamtchlor [Gew.-%] | - | - | - | 0,034 |

**Tabelle 4**

| Verteilungskoeffizienten der Verunreinigungen | | | | |
|---|---|---|---|---|
| Ausgangsschmelze | 1 | 2 | 3 | 4 |
| Verteilungskoeffizient Schwefelsäure | 0,150 | 0,118 | 0,096 | - |
| Verteilungskoeffizient Salpetersäure | - | 0,117 | 0,069 | - |
| Verteilungskoeffizient Methansulfonsäuremethylester | 0,124 | - | - | - |
| Verteilungskoeffizient Gesamtchlor | - | - | - | 0,068 |

### Bezugszeichenliste

- 1: Rohalkansulfonsäure
- 2: erster Prozessschritt
- 3: Leichtsiederabtrennung
- 4: Einsatzstoffe
- 5: Leichtsieder enthaltender Stoffstrom
- 7: Alkansulfonsäure, Schwersieder und Reste an Leichtsiedern enthaltender Stoffstrom
- 9: Schmelzekristallisation
- 10: Mutterlauge
- 11: Wäscher
- 12: Suspension
- 13: Produkt
- 14: Fest-Flüssig-Trennung
- 15: Rückführung in die Schmelzekristallisation
- 16: Waschflüssigkeit
- 17: Rückführung in die Destillationskolonne
- 18: Kristallisationskreis
- 19: Verdampfer, Schwersiederabtrennung
- 20: Destillationskreis
- 21: Brüden
- 22: Schwersiederkreis
- 23: Teilkondensator
- 24: Schwersiederpurge
- 25: Kondensat
- 26: Aufgearbeiteter Schwersiederpurgestrom

## Patentansprüche

1. Verfahren zur Aufreinigung von Alkansulfonsäuren, folgende Schritte umfassend:
(a) Destillation einer Rohalkansulfonsäure (1) enthaltenden Schmelze zur vollständigen oder teilweisen Abtrennung von Leichtsiedern, wobei die Leichtsieder am Kopf einer Destillationskolonne (3) oder eines einstufigen Verdampfungsapparates abgezogen werden und ein Alkansulfonsäure, Schwersieder und Reste an Leichtsiedern enthaltender Stoffstrom (7) am Sumpf der Destillationskolonne (3) oder des einstufigen Verdampfungsapparates entnommen wird,
(b) Zufuhr des Alkansulfonsäure, Schwersieder und Reste an Leichtsiedern enthaltenden Stromes (7) als Ausgangsschmelze in eine Schmelzekristallisation (9), wobei sich Kristalle der Alkansulfonsäure, von Hydraten der Alkansulfonsäure oder einer Mischung aus beiden suspendiert in Mutterlauge bilden,
(c) Durchführen einer Fest-Flüssig-Trennung, um die Kristalle aus der Mutterlauge abzutrennen,
(d) Gegebenenfalls Waschen der Kristalle, um an den Kristallen anhaftende Mutterlauge zu entfernen.

2. Verfahren gemäß Anspruch 1, **dadurch gekennzeichnet, dass** die Mutterlauge nach Abtrennung der Kristalle in Schritt (c) und/oder die in Schritt (b) anfallende Mutterlauge zumindest teilweise in die Schmelzekristallisation (9) oder in die Destillation in Schritt (a) zurückgeführt wird.

3. Verfahren gemäß Anspruch 1 oder 2, **dadurch gekennzeichnet, dass** die Mutterlauge nach Abtrennung der Kristalle in Schritt (c) und/oder die in Schritt (b) anfallende Mutterlauge zumindest teilweise einer Schwersiederabtrennung (19) zugeführt wird, um Schwersieder aus der Mutterlauge zu entfernen, wobei vorzugsweise die Mutterlauge nach dem Entfernen der Schwersieder in die Destillation in Schritt (a) zurückgeführt wird oder die Mutterlauge nach dem Entfernen der Schwersieder teilkondensiert wird und der kondensierte Anteil in die Destillation in Schritt (a) zurückgeführt wird und der nicht kondensierte Anteil als Leichtsieder aus dem Prozess ausgeschleust wird.

4. Verfahren gemäß einem der Ansprüche 1 bis 3, **dadurch gekennzeichnet, dass** die Alkansulfonsäure Methansulfonsäure ist.

5. Verfahren gemäß einem der Ansprüche 1 bis 4, **dadurch gekennzeichnet, dass** die Leichtsieder Wasser oder Schwefeltrioxid umfassen.

6. Verfahren gemäß einem der Ansprüche 1 bis 5, **dadurch gekennzeichnet, dass** der der Schmelzekristallisation (9) zugeführte Alkansulfonsäure, Schwersieder und Reste an Leichtsiedern enthaltende Stoffstrom neben Wasser beziehungsweise Schwefeltrioxid maximal 6 Gew.-% weitere Verunreinigungen enthält bezogen auf die Gesamtmasse des Alkansulfonsäure, Schwersieder und Reste an Leichtsiedern enthaltenden Stoffstroms.

7. Verfahren gemäß einem der Ansprüche 1 bis 6, **dadurch gekennzeichnet, dass** die Schmelzekristallisation (9) bei einer Temperatur im Bereich von -15 bis 19°C durchgeführt wird.

8. Verfahren gemäß einem der Ansprüche 1 bis 7, **dadurch gekennzeichnet, dass** der Alkansulfonsäure, Schwersieder und Reste an Leichtsiedern enthaltende Stoffstrom (7) mindestens 76 Mol.-% Alkansulfonsäure enthält bezogen auf die Gesamtmenge an Alkansulfonsäure und Wasser im Alkansulfonsäure, Schwersieder und Reste an Leichtsiedern enthaltenden Stoffstrom.

9. Verfahren gemäß einem der Ansprüche 1 bis 8, **dadurch gekennzeichnet, dass** der Alkansulfonsäure, Schwersieder und Reste an Leichtsiedern enthaltende Stoffstrom (7) mindestens 87 mol % Alkansulfonsäure enthält bezogen auf die Gesamtmenge an Alkansulfonsäure und Schwefeltrioxid im Alkansulfonsäure, Schwersieder und Reste an Leichtsiedern enthaltenden Stoffstrom.

10. Verfahren gemäß Anspruch 8 oder 9, **dadurch gekennzeichnet, dass** die Schmelzekristallisation (9) bei einer Temperatur im Bereich von -10 bis 19°C durchgeführt wird.

11. Verfahren gemäß einem der Ansprüche 1 bis 7, **dadurch gekennzeichnet, dass** der Alkansulfonsäure, Schwersieder und Reste an Leichtsiedern enthaltende Stoffstrom (7) 31 bis 75 Mol.-% Alkansulfonsäure enthält bezogen auf die Gesamtmenge an Alkansulfonsäure und Wasser im Alkansulfonsäure, Schwersieder und Reste an Leichtsiedern enthaltenden Stoffstrom, wobei die Schmelzekristallisation (9) vorzugsweise bei einer Temperatur im Bereich von -15 bis 12°C durchgeführt wird.

12. Verfahren gemäß einem der Ansprüche 1 bis 11, **dadurch gekennzeichnet, dass** die Kristalle in Schritt (d) mit geschmolzenem Kristallisat gewaschen werden.

13. Verfahren gemäß Anspruch 12, **dadurch gekennzeichnet, dass** das geschmolzene Kristallisat zum Waschen der Kristalle eine Temperatur aufweist, die 0,1 bis 15°C oberhalb der Erstarrungstemperatur des Alkansulfonsäure-haltigen Kristallisats liegt.

14. Verfahren gemäß einem der Ansprüche 3 bis 13, **dadurch gekennzeichnet, dass** der der Destillation entnommene, Alkansulfonsäure, Schwersieder und Reste an Leichtsiedern enthaltende Strom (7) vor Zugabe in die Schmelzekristallisation (9) abgekühlt und die Mutterlauge, die in die Destillation zurückgeführt wird, geheizt wird, wobei vorzugsweise Wärme von dem abzukühlenden, Alkansulfonsäure, Schwersieder und Reste an Leichtsiedern enthaltenden Strom (7) an die zu heizende Mutterlauge übertragen wird.

15. Verfahren gemäß einem der Ansprüche 1 bis 14, **dadurch gekennzeichnet, dass** die Schmelzekristallisation eine Suspensionskristallisation oder eine Schichtkristallisation ist.

## Claims

1. A process for purifying alkanesulfonic acids which comprises the steps of:
(a) distilling a melt comprising crude alkanesulfonic acid (1) to completely or partly remove low boilers, wherein the low boilers are drawn off at the top of a distillation column (3) or of a one-stage evaporation apparatus and a material stream (7) comprising alkanesulfonic acid, high boilers and residual low boilers is withdrawn at the bottom of the distillation column (3) or of the one-stage evaporation apparatus,
(b) sending the stream (7) comprising alkanesulfonic acid, high boilers and residual low boilers into a melt crystallization (9) as the starting melt to form crystals of the alkanesulfonic acid, of hydrates of the alkanesulfonic acid or of a mixture of both suspended in mother liquor,
(c) performing a solid-liquid separation to remove the crystals from the mother liquor,
(d) optionally washing the crystals to remove mother liquor adhering to the crystals.

2. The process according to claim 1, wherein the mother liquor after removal of the crystals in step (c) and/or the mother liquor generated in step (b) is/are at least partly recycled into the melt crystallization (9) or into the distillation in step (a).

3. The process according to claim 1 or 2, wherein the mother liquor after removal of the crystals in step (c) and/or the mother liquor generated in step (b) is/are at least partly sent into a high boilers removal (19) to remove high boilers from the mother liquor, where preferably the mother liquor after removal of the high boilers is recycled into the distillation in step (a) or the mother liquor after removal of the high boilers is partially condensed and the condensed portion is recycled into the distillation in step (a) and the uncondensed portion is discharged from the process as low boilers.

4. The process according to any of claims 1 to 3, wherein the alkanesulfonic acid is methanesulfonic acid.

5. The process according to any of claims 1 to 4, wherein the low boilers comprise water or sulfur trioxide.

6. The process according to any of claims 1 to 5, wherein the material stream comprising alkanesulfonic acid, high boilers and residual low boilers sent to the melt crystallization (9) comprises in addition to water or sulfur trioxide not more than 6 wt% of further impurities based on the total mass of the material stream comprising alkanesulfonic acid, high boilers and residual low boilers.

7. The process according to any of claims 1 to 6, wherein the melt crystallization (9) is carried out at a temperature in the range from -15°C to 19°C.

8. The process according to any of claims 1 to 7, wherein the material stream (7) comprising alkanesulfonic acid, high boilers and residual low boilers comprises at least 76 mol% of alkanesulfonic acid based on the total amount of alkanesulfonic acid and water in the material stream comprising alkanesulfonic acid, high boilers and residual low boilers.

9. The process according to any of claims 1 to 8, wherein the material stream (7) comprising alkanesulfonic acid, high boilers and residual low boilers comprises at least 87 mol% of alkanesulfonic acid based on the total amount of alkanesulfonic acid and sulfur trioxide in the material stream comprising alkanesulfonic acid, high boilers and residual low boilers.

10. The process according to claim 8 or 9, wherein the melt crystallization (9) is carried out at a temperature in the range from -10°C to 19°C.

11. The process according to any of claims 1 to 7, wherein the material stream (7) comprising alkanesulfonic acid, high boilers and residual low boilers comprises 31 to 75 mol% of alkanesulfonic acid based on the total amount of alkanesulfonic acid and water in the material stream comprising alkanesulfonic acid, high boilers and residual low boilers, where the melt crystallization (9) is preferably carried out at a temperature in the range from -15°C to 12°C.

12. The process according to any of claims 1 to 11, wherein step (d) comprises washing the crystals with molten crystallizate.

13. The process according to claim 12, wherein the molten crystallizate for washing the crystals has a temperature 0.1°C to 15°C above the solidification temperature of the alkanesulfonic-acid-containing crystallizate.

14. The process according to any of claims 3 to 13, wherein the stream (7) comprising alkanesulfonic acid, high boilers and residual low boilers withdrawn from the distillation is cooled before being sent to the melt crystallization (9) and the mother liquor which is recycled into the distillation is heated, where preferably heat from the stream (7) comprising alkanesulfonic acid, high boilers and residual low boilers which is to be cooled is transferred to the mother liquor which is to be heated.

15. The process according to any of claims 1 to 14, wherein the melt crystallization is a suspension crystallization or a layer crystallization.

## Revendications

1. Procédé de purification d'acides alcane-sulfoniques, comprenant les étapes suivantes :
(a) la distillation d'une masse fondue contenant un acide alcane-sulfonique brut (1) pour la séparation totale ou partielle de composants de point d'ébullition faible, les composants de point d'ébullition faible étant soutirés à la tête d'une colonne de distillation (3) ou d'un appareil d'évaporation à un niveau, et un courant de matière (7) contenant l'acide alcane-sulfonique, les composants de point d'ébullition élevé et les résidus de composants de point d'ébullition faible étant soutiré au fond de la colonne de distillation (3) ou de l'appareil d'évaporation à un niveau,
(b) l'introduction du courant (7) contenant l'acide alcane-sulfonique, les composants de point d'ébullition élevé et les résidus des composants de point d'ébullition faible en tant que masse fondue de départ dans une cristallisation de masse fondue (9), des cristaux de l'acide alcane-sulfonique, d'hydrates de l'acide alcane-sulfonique ou d'un mélange des deux se formant sous forme suspendue dans la liqueur mère,
(c) la réalisation d'une séparation solide-liquide, afin de séparer les cristaux de la liqueur mère,
(d) éventuellement le lavage des cristaux, afin d'éliminer la liqueur mère adhérant aux cristaux.

2. Procédé selon la revendication 1, **caractérisé en ce que** la liqueur mère après la séparation des cristaux à l'étape (c) et/ou la liqueur mère formée à l'étape (b) sont au moins partiellement recyclées dans la cristallisation de masse fondue (9) ou dans la distillation de l'étape (a).

3. Procédé selon la revendication 1 ou 2, **caractérisé en ce que** la liqueur mère après la séparation des cristaux à l'étape (c) et/ou la liqueur mère formée à l'étape (b) sont au moins partiellement introduites dans une séparation des composants de point d'ébullition élevé (19), afin de séparer les composants de point d'ébullition élevé de la liqueur mère, la liqueur mère après l'élimination des composants de point d'ébullition élevé étant de préférence recyclée dans la distillation de l'étape (a) ou la liqueur mère après l'élimination des composants de point d'ébullition élevé étant partiellement condensée, et la fraction condensée étant recyclée dans la distillation de l'étape (a), et la fraction non condensée étant évacuée du procédé en tant que composant de point d'ébullition faible.

4. Procédé selon l'une quelconque des revendications 1 à 3, **caractérisé en ce que** l'acide alcane-sulfonique est l'acide méthane-sulfonique.

5. Procédé selon l'une quelconque des revendications 1 à 4, **caractérisé en ce que** les composants de point d'ébullition faible comprennent de l'eau ou du trioxyde de soufre.

6. Procédé selon l'une quelconque des revendications 1 à 5, **caractérisé en ce que** le courant de matière contenant l'acide alcane-sulfonique, les composants de point d'ébullition élevé et les résidus des composants de point d'ébullition faible introduit dans la cristallisation de masse fondue (9) contient, outre l'eau ou le trioxyde de soufre, au plus 6 % en poids d'autres impuretés, par rapport à la masse totale du courant de matière contenant l'acide alcane-sulfonique, les composants de point d'ébullition élevé et les résidus des composants de point d'ébullition faible.

7. Procédé selon l'une quelconque des revendications 1 à 6, **caractérisé en ce que** la cristallisation de masse fondue (9) est réalisée à une température dans la plage allant de -15 à 19 °C.

8. Procédé selon l'une quelconque des revendications 1 à 7, **caractérisé en ce que** le courant de matière contenant l'acide alcane-sulfonique, les composants de point d'ébullition élevé et les résidus des composants de point d'ébullition faible (7) contient au moins 76 % en moles d'acide alcane-sulfonique, par rapport à la quantité totale d'acide alcane-sulfonique et d'eau dans le courant de matière contenant l'acide alcane-sulfonique, les composants de point d'ébullition élevé et les résidus des composants de point d'ébullition faible.

9. Procédé selon l'une quelconque des revendications 1 à 8, **caractérisé en ce que** le courant de matière contenant l'acide alcane-sulfonique, les composants de point d'ébullition élevé et les résidus des composants de point d'ébullition faible (7) contient au moins 87 % en moles d'acide alcane-sulfonique, par rapport à la quantité totale d'acide alcane-sulfonique et de trioxyde de soufre dans le courant de matière contenant l'acide alcane-sulfonique, les composants de point d'ébullition élevé et les résidus des composants de point d'ébullition faible.

10. Procédé selon la revendication 8 ou 9, **caractérisé en ce que** la cristallisation de masse fondue (9) est réalisée à une température dans la plage allant de -10 à 19 °C.

11. Procédé selon l'une quelconque des revendications 1 à 7, **caractérisé en ce que** le courant de matière contenant l'acide alcane-sulfonique, les composants de point d'ébullition élevé et les résidus des composants de point d'ébullition faible (7) contient 31 à 75 % en moles d'acide alcane-sulfonique, par rapport à la quantité totale d'acide alcane-sulfonique et d'eau dans le courant de matière contenant l'acide alcane-sulfonique, les composants de point d'ébullition élevé et les résidus des composants de point d'ébullition faible, la cristallisation de masse fondue (9) étant de préférence réalisée à une température dans la plage allant de -15 à 12 °C.

12. Procédé selon l'une quelconque des revendications 1 à 11, **caractérisé en ce que** les cristaux sont lavés avec du cristallisat fondu à l'étape (d).

13. Procédé selon la revendication 12, **caractérisé en ce que** le cristallisat fondu pour le lavage des cristaux présente une température qui se situe 0,1 à 15 °C au-dessus de la température de solidification du cristallisat contenant de l'acide alcane-sulfonique.

14. Procédé selon l'une quelconque des revendications 3 à 13, **caractérisé en ce que** le courant (7) contenant l'acide alcane-sulfonique, les composants de point d'ébullition élevé et les résidus des composants de point d'ébullition faible, soutiré de la distillation, est refroidi avant l'ajout dans la cristallisation de masse fondue (9), et la liqueur mère qui est recyclée dans la distillation est chauffée, la chaleur du courant (7) à refroidir, contenant l'acide alcane-sulfonique, les composants de point d'ébullition élevé et les résidus des composants de point d'ébullition faible, étant de préférence transférée à la liqueur mère à chauffer.

15. Procédé selon l'une quelconque des revendications 1 à 14, **caractérisé en ce que** la cristallisation de masse fondue est une cristallisation en suspension ou une cristallisation en couches.
